# EUROPEAN PATENT SPECIFICATION

(11) **EP 1 894 572 B1**
(45) Date of publication and mention of the grant of the patent: **04.12.2013**
(21) Application number: 06757103.4
(22) Date of filing: 07.06.2006
(51) Int. Cl.: A61K 38/23, A61P 25/00, A61P 29/00

(54) **THERAPEUTIC AGENT AND/OR PROPHYLACTIC AGENT FOR NEUROGENIC PAIN**
THERAPEUTISCHER UND/ODER PROPHYLAKTISCHER WIRKSTOFF GEGEN NEUROGENE SCHMERZEN
AGENT THERAPEUTIQUE ET/OU AGENT PROPHYLACTIQUE POUR UNE DOULEUR NEUROGENE

(30) Priority: 09.06.2005 JP 2005170165
(43) Date of publication of application: 05.03.2008
(73) Proprietor: Asahi Kasei Pharma Corporation, Tokyo 101-8481 (JP)
(72) Inventor: ITO, Akitoshi, Tokyo 1008440 (JP); TAKEDA, Mineko, Tokyo 1008440 (JP)
(74) Representative: Forstmeyer, Dietmar
(86) International application number: PCT/JP2006/311396
(87) International publication number: WO 2006/132261

(56) References cited:
- JP-A- 11 504 011
- QUATRARO A. ET AL.: 'Calcitonin in painful diabetic neuropathy' THE LANCET vol. 339, 1992, pages 746 - 747, XP008073556
- KINGERY W.S.: 'A critical review of controlled clinical trials for peripheral neuropathic pain and complex regional pain syndromes' PAIN vol. 73, no. 2, 1997, pages 123 - 139, XP002119434
- APPELBOOM T.: 'Calcitonin in reflex sympathetic dystrophy syndrome and other painful conditions' BONE vol. 30, no. 5, SUPPL. 1, 2002, pages 84S - 86S, XP003005021
- YOSHIMURA M.: 'Analgesic mechanism of calcitonin' JOURNAL OF BONE AND MINERAL METABOLISM vol. 18, no. 4, 2000, pages 230 - 233, XP003005022

## Description

### Field of the Invention

The present invention relates to a calcitonin preparation having a quick-acting analgesic effect on neuropathic pain.

### Background of the Invention

Neuropathic pain is an intractable pain caused by dysfunction in the peripheral or central nervous system. Neuropathic pain is initiated by neuropathy caused by metabolic disorders such as trauma, infection, cancer, ischemia, diabetes and the like. Though the onset mechanism is not very clear, abnormal continuous firing of the sensory nerve and the like are thought to be the causes. Typical symptoms of neuropathic pain include allodynia, hyperalgesia, hyperesthesia and the like. These symptoms manifest characteristic pains described as "burning", "stinging", "electroshock-like" and the like. It is known that analgesics effective for usual nociceptive pain, particularly narcotic analgesics, and the like are not so effective for neuropathic pain (Non-patent Literature 1). Conventionally known analgesics include central opioid-based analgesics typified by morphine, nonsteroidal anti-inflammatory drugs (NSAIDs) typified by indomethacin, and the like. These drugs, however, have a poor effect on neuropathic pain and, like NSAIDs on acute pain, just blocking the production of an algesic substance cannot remove the pain.

It is known that while morphine has a strong analgesic action on nociceptive pain, it does not show a sufficient effect on neuropathic pain (Non-patent Literature 2). The reason that morphine is not effective on neuropathic pain is believed to be due to the decrease of opioid receptors (Non-patent Literature 3) caused by the functional and morphological changes in the nerve caused by neuropathy.

Therefore, various factors are believed to be intricately interrelated with the onset of neuropathic pain. Current treatment methods are neurosurgical treatments such as nerve block, spinal epidural electric stimulation and the like (Non-patent Literature 4), lumbar intraspinal administration of baclofen (Non-patent Literature 5), and the like.

Further, in pain therapy, continuation of pain causes a plastic change in the neural network, shifting to intractable pain. This is considered problematical, thus, early pain control is one of the most important tasks (Non-patent Literature 6). A highly safe drug capable of long-term pain control until nervous disorders are cured has been desired.

On the other hand, a calcitonin preparation is used for treatment of hypercalcemia and osteoporosis, and acts via a calcitonin receptor of an osteoclast.
Calcitonin receptors on an osteoclast cause down-regulation when calcitonin is allowed to act continuously, which is feared to result in the inability of serum calcium concentration control. Since no disjunction of effective concentration between variation in serum calcium and effective concentration of analgesic action has been reported thus far, a calcitonin preparation is administered intermittently, daily or once per week.

It is known up to now that calcitonin has an analgesic action on a certain kind of pain, and it is also known that its action mechanism differs depending on the kind of pain. For example, for inflammatory pain, a prostaglandin production suppressing action and correlation of intracerebral ascending serotonin nerve system are reported. Regarding pain of bone metastasis cancer, an inhibitory action for a bone resorption at a bone metastasis site and an action via secretion of β-endorphin (endogenous opioid) are known. However, just as NSAIDs and morphine are not very effective, these mechanisms are not expected to present substantially effective action on neuropathic pain. In neuropathic pain caused by peripheral nerve disorders, it is believed that abnormal continuous firing of the sensory nerve, its threshold decrease, and hyperalgesia are occurring. These phenomena are reported to be induced by altered gene expressions such as voltage-dependent sodium channel in the sensory nerve, and believed to be a pathological condition independent of other pains (Non-patent Literature 7, Non-patent Literature 8).

There is a clinical report that intermittent administration of a calcitonin preparation is effective on various neuropathic pains, but a longer period of time was necessary for the effect to manifest, and also, its effect is substantially utterly insufficient (Non-patent Literature 9, Non-patent Literature 10).

As described above, in treatment of neuropathic pain, a safe and quick-acting effective therapeutic method is not established and the development of a new therapy is eagerly desired.
Non-patent Literature 1: The Lancet 353, 1959-1966, 1999
Non-patent Literature 2: Igaku no Ayumi, 189 (10), 751-755, 1999
Non-patent Literature 3: Saishin Nou to Shinkeikagaku Series, vol. 6, Itami no Shinkeikagaku, Medical View, p97, 1997
Non-patent Literature 4: Igaku no Ayumi, 189(10), 757-762
Non-patent Literature 5: Kinoteki Noushinkeikagaku 33 45-49, 1994
Non-patent Literature 6: Kumazawa Takao; Itami ha Hizumu. Nou wo shiru. edited by Hisano Takashi, Shujunsha, 106-116, 1999
Non-patent Literature 7: Boucher TJ et al: Potent analgesic effects of GDNF in neuropathic pain states. Science, 2000, 290, 124-127
Non-patent Literature 8: Hong S et al, Early painful diabetic neuropathy is associated with differential changes in tetrodotoxin-sensitive and resistant sodium channels in dorsal root ganglion neurons in the rat. J Biol Chem. 2004 Jul 9; 279(28): 29341-50
Non-patent Literature 9: Wade S et al: A critical reviews of controlled clinical trials for peripheral neuropathic pain and complex regional pain syndromes, PAIN, USA, 1997, 73, 123-139
Non-patent Literature 10: Antonio Quatraro, calcitonin in painful diabetic neuropathy, THE LANSET, 1992, 339, 746-747

### Disclosure of the Invention

### Problem to Be Solved by the Invention

The object of the present invention is to provide a novel medicine having an excellent quick-acting analgesic action on neuropathic pain.

### Means to Solve the Problem

For attaining the above-mentioned object, a study has been intensively carried out focusing attention on calcitonin, particularly elcatonin. Though it was difficult to develop a substantially effective neuropathic pain treating drug containing calcitonin as an active ingredient, from an action mechanism of an analgesic action of calcitonin and intermittent administration results for neuropathic pain known until now, an action of calcitonin for repairing gene expression of Na channel in sensory nerve (Ito et al., Osteoporosis Japan, 2005, vol. 13, no. 1, 78-80) was found. Therefore, optimization of this analgesic action mechanism has been investigated imagining that calcitonin would have a completely separate novel analgesic pharmacological action mechanism. Specifically, a calcitonin administration method which manifests a quick-acting analgesic action on neuropathic pain without altering the serum calcium value has been intensively studied using a model evaluation system for neuropathic pain, and consequently it has been found, surprisingly, that a quick-acting strong analgesic action is shown when calcitonin, particularly elcatonin is administered continuously, further that the same effect is shown also by continuous administration of dosage without altering serum calcium, leading to the present invention based on these findings.

That is, the present invention relates to
(1) An agent comprising calcitonin as an active ingredient for use in a method of treating and/or preventing neuropathic pain, wherein calcitonin is continuously administered in an amount less than 0.093 mU/kg/second.
(2) The agent for use as in (1), wherein the duration of the continuous administration is 8 hours or more.
(3) The agent for use as in (1) or (2), wherein the neuropathic pain is caused by the compression of the peripheral nerve or the injury of the peripheral nerve.
(4) The agent for use as in any of (1) to (3), wherein the calcitonin is elcatonin.
(5) The agent for use as in any of (1) to (4), wherein the dosage form is a sustained release preparation, a drip preparation or a patch preparation.
(6) The agent for use as in (5), wherein the dosage form is an injection preparation, an implanted preparation, a percutaneous preparation, a transnasal preparation, a transpulmonary preparation or a peroral preparation.

### Effects of the Invention

The present invention provides an agent comprising calcitonin as active ingredient for use in a method of treating and/or preventing neuropathic pain which can reduce patient load and side effects by drug administration and which is more effective and quick-acting.

### Detailed Description of Preferred Embodiments

The present invention will be illustrated specifically below.

As calcitonin useful as an active ingredient of the neuropathic pain treating and/or preventing agent of the present invention, various natural type calcitonins, or peptide analogues thereof and the like are mentioned. Examples of the natural type calcitonin include chicken calcitonin, eel calcitonin, human calcitonin, salmon calcitonin, pig calcitonin and the like, and eel calcitonin and salmon calcitonin (Helv. Chim. Acta (1969), 52(7), 1789-95) are mentioned as preferable examples, eel calcitonin is mentioned as particularly preferable example. In another embodiment, salmon calcitonin may be preferable.

As examples of peptide analogues of natural type calcitonin, mentioned are compounds obtained by chemically modifying a disulfide bond at 1,7-position based on a structure of the natural type calcitonin, and specifically, [ASU 1-7] chicken calcitonin, [ASU 1-7] eel calcitonin (chemical name: 1-butylic acid-7-(L-2-aminobutylic acid)-26-L-aspartic acid-27-L-valine-29-L-alaninecalcitonin described in Japanese Patent Application Publication (JP-B) No. 53-41677; hereinafter referred to also as "elcatonin" in some cases), and the like are mentioned as preferable examples, and [ASU 1-7] eel calcitonin (elcatonin) is mentioned as a particularly preferable example.

As calcitonin useful as an active ingredient of the agent for treating and/or preventing neuropathic pain of the present invention, elcatonin or salmon calcitonin is particularly preferable, and elcatonin is most preferable.

As the active ingredient of the agent for treating and/or preventing neuropathic pain of the present invention, salts of calcitonin useful as the active ingredient of the agent for treating and/or preventing neuropathic pain of the present invention, with pharmacologically acceptable acids may be used. As the salts with pharmacologically acceptable acids, for example, salts with inorganic acids such as hydrochloric acid, sulfuric acid and the like, salts with organic acids such as acetic acid, tartaric acid, succinic acid, malic acid and the like are mentioned as suitable examples.

Further, a medicinal composition can also be produced using pharmacologically acceptable carriers according to an ordinary method.

Continuous administration of the present invention means an administration method of releasing a drug into a body continuously for a certain duration or more, and the administration route is not limited providing it is systemic administration or local administration to peripheral tissue, and for example, administration using a device such as an infusion pump, transfusion pump and the like or manual administration, and sustained-release preparations using as a carrier a polymer decomposed in a body, are mentioned. Further included is an administration method of controlling the administration amount of calcitonin by elongating efficacy by polyethylene glycosylation and the like. In contrast, intermittent administration means administering or releasing a drug into a body once or multiple times with an interval of certain time, not in continuous fashion.

The dosage form suitable for continuous administration of calcitonin includes a sustained release preparation, a drip preparation, a patch preparation and the like. The patch preparation includes also someone in which a fine needle is provided on a surface to be pasted to skin, and a drug oozes from the needle. In detail, for example, a drip preparation or continuous injection preparation of injection liquid and sustained-release preparation are mentioned. As the dosage form of a sustained-release preparation, exemplified are an injection preparation, implant preparation, percutaneous preparation, transnasal preparation, transpulmonary preparation, peroral preparation and the like. More specifically, exemplified as the sustained release preparation are sustained release injection preparations in which calcitonin as a drug is contained in a biodegradable or non-biodegradable fine particle carrier such as microcapsule, emulsion, liposome, fat and oil, poly (lactic-co-glycolic asid), polylactic acid, hydroxyapatite and the like, the fine particles are administered in the form of injection by subcutaneous, intracutaneous, intramuscular route, and the like, and the drug is released continuously in an organism; implant preparations in which a drug is allowed to be contained in a biodegradable or non-biodegradable substance such as silicone and the like to give a solid material in the form of pellet, needle and the like, and the solid material is implanted under skin and the like; percutaneous preparations in which permeability of a drug is enhanced using an absorption promoter or electric energy, or physically such as by skin scratch and the like, a patch or tape containing a drug is formed and the drug is allowed to be absorbed continuously from skin; percutaneous preparations of type in which a fine needle is provided on a surface to be pasted to skin, and a drug oozes from the needle under control and of type in which a drug is coated on a fine needle; further, transnasal preparations and transpulmonary preparations in which microcapsules and nano particles and the like showing controlled decomposition and release of a drug are used and the drug is allowed to be absorbed continuously via mucosa and the like, peroral preparations such as tablet, capsule and the like prepared by adding a mucoadhesive polymer to gastrointestinal tract, to these transnasal preparations and transpulmonary preparations; peroral preparations using liposome and the like.

The continuous administration time in the present invention is preferably 8 hours or more, further preferably 12 hours or more, particularly preferably 16 hours or more. As the administration period, 2 months or less are exemplified, 1 month or less are preferable, and 2 weeks or less are further preferable.

From Comparative Example 1 described later, it has been considered that an analgesic action by intermittent administration of calcitonin is affected by accumulation of an effect in each administration. The blood retention time by intermittent administration is about 2 hours. Therefore, also included in the range of the present invention is an administration method in which continuous administration of 2 hours or more and less than 8 hours is repeated and accumulated duration of continuous administration within 24 hours is 8 hours or more.

An analgesic effect of calcitonin once manifested on neuropathic pain continues for at least one week even during the drug withdrawal period, thus, its effect can continue by adding intermittent administration after completion of continuous administration. There is also a therapeutic method ad libitum combining continuous administration and intermittent administration.

Neuropathic pain means an abnormal condition of pain perception in which, due to a cause such as trauma, compression, infection, ischemia and the like, or metabolic disturbances such as diabetes and the like, neuropathy occurs in which nerve, plexus or perineural soft tissue is injured or degenerated, leading to continuation of lowering of pain threshold and the like due to some dysfunction caused by the neuropathy. Examples thereof include, but not limited to, spontaneous pain, pain threshold lowering (pain perception against mechanical stimulation), hyperalgesia (excess response to harmful stimulation) and hyperesthesia (excess response to contact, called also allodynia). Further, numbness and paralysis may accompany in some cases.

As diseases of neuropathic pain induced by a disorder of the peripheral nerve, neuropathic pains due to the compression of the peripheral nerve and injury of the peripheral nerve are mentioned, and particularly, diabetic neuropathy, entrapment (compression) peripheral neuropathy, spinal canal stenosis, disk herniation, carpal tunnel syndrome, reflex sympathetic dystrophy (RSD), shoulder-hand syndrome, CRPS (complex regional pain syndrome) type I, CRPS type II, postherpetic neuralgia, neuropathy due to HIV, atypical facial pain after exodontia and the like, trigeminal neuralgia and the like are mentioned. Further mentioned are postoperative pain, rheumatoid arthritis, osteoarthtiris and the like showing pain remaining also after administration of an anti-inflammatory analgesic. Furthermore, fibromyalgia, intractable regional pain syndrome, pain accompanying neuropathy such as idiopathic or posttraumatic neuropathy and mononeuritis, and neuropathic pain caused by the invasion of cancer into a nerve, are mentioned. Still further, low back pain, stiffness in the shoulder, frozen shoulder and the like showing pain remaining also after administration of an anti-inflammatory analgesic such as NSAIDs and the like are also mentioned.

Neuropathic pain caused by compression of the peripheral nerve means neuropathic pain induced by entrapment (compression) peripheral neuropathy, spinal canal stenosis, disk herniation, carpal tunnel syndrome, and the like. Entrapment (compression) peripheral neuropathy include pains ascribable to cervical spondylosis, cubital tunnel syndrome, cervical spondylosis deformans, ossification of posterior longitudinal ligament, cervical sprain, spondylosis, spondylolysis, spondylolisthesis, spondylolytic spondylolisthesis and the like.

The peripheral nerve means the sensory nerve, the motor nerve, the sympathetic nerve, the parasympathetic nerve, and the like, and the central nerve means the brain and the spinal cord nerve.

Neuropathic pain induced by peripheral neuropathy includes, but not limited to, diseases showing spontaneous pain, pain threshold decrease, hyperalgesia and allodynia in peripheral tissues. Further, neurogenic intermittent claudication, namely, augmentation of pain caused by addition of congestion load to peripheral neuropathy, is also included. Further, numbness and paralysis may accompany in some cases.

Neuropathy may be a mononeuropathy or polyneuropathy. Treatment effect is an effect by which neuropathic pain is treated by administering a drug after a nerve has been injured. More specifically, it is an effect of treating pain by returning the lowered pain threshold up to the normal value.

The amount of calcitonin sufficient for manifestation of an effect of treating neuropathic pain by continuous administration means an amount by which a neuropathic pain treatment effect is manifested by continuous administration to a patient or animal suffering from neuropathic pain. Preferably, it is a concentration that does not alter serum calcium values.

Alteration of serum calcium values means statistically no significant change in comparison with values before initiation of administration or in vehicle administration, and transient change occurring within 24 hours from initiation of administration is permissible providing it is not over a normal range of serum calcium values. In the present invention, serum calcium values are replaceable by blood calcium values or plasma calcium values.

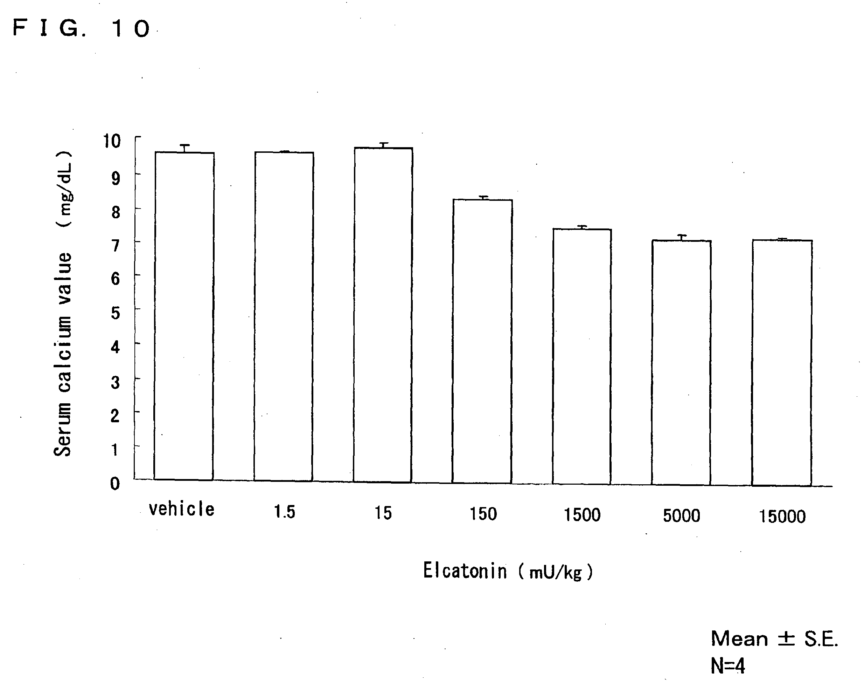

As the dose of elcatonin that does not alter serum calcium values in humans, less than 0. 093 mU/kg/second are exemplified. Namely, in subcutaneous administration of calcitonin 400 U/man/day mixed into morphine continuous administration in a bone metastasis cancer patient, a significant decrease in serum calcium is recognized (J. Pain and Symptom Management, 1999, vol. 18, No. 5, p. 323-330). On the other hand, in drip intravenous administration of 40 U/man/5 hours in a renal osteopathy patient, no significant decrease in serum calcium is recognized (Shinyaku to Rinsho, 1985, vol. 34, no. 4, p. 587-592). Therefore, the calcitonin dose altering serum calcium values in humans will be reasonably estimated to be 0.093 mU/kg/second assuming the human body weight is 50 kg. Further, assuming that the drip intravenous dose is two-thirds of the intramuscular or subcutaneous dose, the subcutaneous dose of elcatonin is further preferably equal to or less than 0.067 mU/kg/second. Test Example 1 described later checks the threshold of the elcatonin dose that does not alter serum calcium values in the case of a single injection of elcatonin to a normal rat (subcutaneous injection over a period of 10 seconds), and as a result, serum calcium values change from the values in the case of administration of elcatonin more than 150 mU/kg, as shown in Fig. 10. In other words the dose of 15 mU/kg/second is estimated to be the serum calcium alteration threshold.

The dose in the case of continuous administration for 1 week or more is preferably between 0.75 mU/kg/week to 75 U/kg/week as shown in Example 3 for rats. Further, as shown in Example 2, administration at 0.075 mU/kg/week to 0.75 mU/kg/week may be preferable in some embodiments. Furthermore, as shown in Example 4, administration at 75 U/kg/week to 400 U/kg/week may be preferable in some embodiments. It is reported that the effective dose in intermittent administration for rat neuropathic pain is 5 U/kg, and in the case of humans, 10 U/man (Shinyaku to Rinsho, 1990, vol. 39, no. 2, p. 130-136). Therefore, the effective dose for neuropathic pain in rats is estimated to be 25-fold higher than in humans assuming that the human body weight is 50 kg. Therefore, the lower limit in humans is preferably 0.003 mU/kg/week or more, more preferably 0.01 mU/kg/week or more, further preferably 0.03 mU/kg/week or more. The upper limit in humans is an amount that does not alter serum calcium values, that is, preferably less than 56 U/kg/week, more preferably 40 U/kg/week or lower, further preferably 18 U/kg/week or lower, most preferably 3 U/kg/week or lower. the dose within this range result in approximately constant analgesia, and hypoalgesia caused by overdose does not occur, thus, dose variation of is permissible in this range.

In another aspect of the present invention, the calcitonin dose per unit time (second) in continuous administration is preferably not more than one-hundredth of the serum calcium alteration threshold dose per unit time (second) of calcitonin. Not bound by a theory, a dosage of 15 mU/kg/second is estimated, based on Test Example 1, Fig. 10, to be a serum calcium-altering threshold, in single injection of calcitonin to rats, as described above. The maximum effective dose of a neuropathic pain treatment by continuous administration in Example 3 is 75 U/kg/week (0.124 mU/kg/second, in terms of second). That is, 0. 124 mU/kg/second is divided by 15 mU/kg/second to give a value of about one-hundredth. It is more preferably one-thousandth or less, further preferably one-five thousandth or less, most preferably one-fifteen thousandth or less. One-twenty thousandth or less are preferable in some embodiments.

In humans, the calcitonin dose per unit time (second) in continuous administration is preferably one-fourth or less of the serum calcium alteration threshold dose per unit time (second) of calcitonin. Not bound by a theory, a dose of 0.093 mU/kg/second is estimated to be the serum calcium alteration threshold in continuous administration of calcitonin to humans, as described above. The maximum effective dose of a neuropathic pain treatment is estimated to be 16 U/kg/week (0.0265 mU/kg/second, in terms of second) in humans which is obtained by dividing 400 U/kg/week by 25, from the result of continuous administration in Example 4. That is, 0.0265 mU/kg/second is divided by 0.093 mU/kg/second to give a value of about one-fourth. It is preferably one-nineteenth or less, more preferably one-hundredth or less, further preferably one-thousandth or less, most preferably one-ten thousandth or less. One-twenty thousandth or less are preferable in some embodiments.

The above-mentioned dosage are examples in the case of subcutaneous and intramuscular administration, and the dosage should be appropriately controlled depending on pharmacokinetics of an administration route such as intravenous, percutaneous, transnasal, transpulmonary, peroral and the like.

Of course, specific dosage should be appropriately controlled depending on the body weight, the sex, the severity of symptoms and the like, and in no case should the patient's serum calcium values be altered.

A method of treating neuropathic pain, comprising a continuous administration for 8 hours or more of a calcitonin preparation in a dose of not lower than an amount necessary for effective therapy in neuropathic pain state and less than a dose that alters serum calcium values is also within the range of the present invention.

Examples of the dosage form of a medicine suitable in the present invention include sustained release preparations, for example, sustained release preparations by subcutaneous injection, intramuscular injection, intravenous injection or intraperitoneal injection, preferably, sustained release preparations by subcutaneous injection or intramuscular injection, drip preparations, patch preparations, and the like. As the sustained release preparations by subcutaneous injection, intramuscular injection, intravenous injection or intraperitoneal injection, exemplified are those prescribed so that calcitonin is used in the above-mentioned dosage, using microspheres using a biodegradable excipient such as poly(D,L-lactide-co-glycolide)polymer as disclosed in Japanese Patent Application National Publication (Laid-Open) No. 11-501027, for example. As the drip preparation, exemplified are those in which a medicine in preparations disclosed in "Nippon Rinsho, vol. 59, No. 9, p. 1789-1793, 2001" is replaced by calcitonin and prescription is so made as to give the above-mentioned dose of calcitonin. Further, since a drip preparation has a possibility of problems of adsorption on a drip vessel and hospital infection, a pre-filled syringe preparation prepared by filling a syringe pump previously with a drug is mentioned as a preferable example. The patch preparation includes a patch preparation of type in which a fine needle is provided on a surface to be pasted to skin, and a drug oozes from the needle, and for example, exemplified are those patch preparations disclosed in Japanese Patent Application National Publication (Laid-Open) No. 2004-528900 in which prescription is so made as to give the above-mentioned dose of calcitonin. As the percutaneous preparation, exemplified are those containing an absorption promoter such as n-octyl-β-D-glucopyranoside and the like and a protease inhibitor such as pestatin and the like as disclosed in Japanese Patent No. 3054175 in which prescription is so made as to give the above-mentioned dosage amount of calcitonin. Further, as examples of the transpulmonary preparation, mentioned are preparations using nanosphere obtained by coating a core part of a poly(lactic-co-glycolic acid) as a biodegradable polymer with chitosan as a mucoadhesive polymer as disclosed in Japanese Patent Application Publication (JP-A) No. 2000-143533, in which prescription is so made as to give the above-mentioned dosage amount of calcitonin. Further, examples of the peroral preparations include those using nanosphares coated with chitosan as disclosed in Japanese Patent Application Publication (JP-A) No. 11-116499, in which prescription is so made as to give the above-mentioned dosage amount of calcitonin. Further, a transnasal preparation can also be obtained by aseptically filling a mechanical spray device applied vial for intranasal administration exemplified in Japanese Patent Application Publication (JP-B) No. 7-8806 with a solution using nanosphere coated with chitosan in which prescription is so made as to give the above-mentioned dosage of calcitonin. Further, a preparation adhering to a gastrointestinal tract and having improved absorptivity and persistency using liposome coated with dodecylated chitosan as disclosed in "Pharmazie, 61(2), 106-111, 2006" which is prescribed to provide the above-mentioned dose of calcitonin is also mentioned as an example of the peroral preparation. Needless to say, the preparation is not limited to these examples.

In addition, exemplified as the sustained release preparation is that obtained by filling a mini osmotic pump (2002) manufactured by ALZA with calcitonin so as to give the above-mentioned dosage amount as shown in Example 1 described later, and this can be implanted subcutaneously at neck.

The continuous administration of the present invention may be simultaneously used with the intermittent administration of calcitonin or an osteoporosis therapeutic agent for a patient or animal with both osteoporosis and neuropathic pain. For a patient or animal with neuropathic pain alone, or with neuropathic pain complicated by a chronic pain such as cancer pain, inflammatory pain, rheumatoid arthritis, osteoarthritis, low back pain, stiffness in the shoulder, frozen shoulder, fibromyalgia and the like, psychogenic pain and the like, the continuous administration of the present invention may be concomitantly used with opioids, cannabinoid receptor agonist, anti-inflammatory analgesic, antidepressant, nerve block agent, blood flow improving agents such as PGE1 derivative and the like, antiepileptics such as gabapentin and the like, steroid hormone, neutotrophic factor, anti-NGF antibody, anti-cytokine antibody, P38MAPK inhibitor, serotonin receptor agonist, acetylcholine receptor agonist, bradykinin receptor agonist, PKC inhibitor, bone resorption suppressing agent, antirheumatic, osteoporosis therapeutic agent, neurotropin, anti-virus agent, noradrenalin receptor agonist, ATP receptor antagonist, vitamin B12 preparation, glutamate receptor agonists such as mGluR1 antagonist and the like, Na channel inhibitor, Ca channel inhibitor, GABA receptor agonist, NMDA antagonist and the like.

Further, use of calcitonin for producing an agent for treating and/or preventing neuropathic pain is also within the range of the present invention.

### Examples

The present invention will be illustrated based on examples and reference examples, but the range of the present invention is not limited to the following examples.

### Example 1

For confirming an effect of continuous administration of elcatonin (ECT) in neuropathic pain by peripheral nerve injury, a chronic constriction injury model (CCI model) described in Namiki et al., Masuikai to Kisokenkyu: Totsu to Chintsu (NANKO DO), p. 37 (2000) as its model animal was improved and studied. Four-week old male SD (IGS) rats was purchased and subjected to preliminary breeding, taming by handling, and domestication for a measuring apparatus of mechanical withdrawal threshold, each for 5 days, then, at 7-weeks old, a sciatic nerve constriction operation or a sham-operation was carried out and the rats were tested.

Under anesthesia with ether, a rat was placed in prone position, and while touching the right femur, the skin was dissected right above it. Biceps femoris was peeled at the center of the femur, and exposed by about 5 mm trying not to injure the sciatic nerve. Using 4-0 blade silk, the sciatic nerve was ligated loosely 4 times in turn from the peripheral side. Thereafter, the fascia and the skin were sutured.

Using withdrawal threshold and body weight on the 8th day after the constriction operation as indices, the rats were divided into a CCI-vehicle group (12 rats), and CCI-ECT 0.75 mU/kg/week, CCI-ECT 7.5 mU/kg/week and CCI-ECT 75 mU/kg/week groups (each 12 rats), by a randomization method (SAS system, version 8.2). On the 11th day after the constriction operation, a mini osmotic pump (2002 model) manufactured by ALZA was filled with a drug to attain the above-mentioned administration amounts, and implanted subcutaneously at neck under ether anesthetization. As a vehicle, 0.02% BSA-containing 0.1 mM sodium acetate buffer (pH 5.5) was used. The flow rate of the mini osmotic pump manufactured by ALZA was constant (0.5 µL/hour), and the dose of elcatonin was controlled by the concentration of elcatonin to be filled. Body weight used for administration was estimated body weight after initiation of administration and calculated by multiplying body weight at start of administration by body weight increase coefficient (1.1) obtained in the preliminary experiment. The concentration of filled elcatonin was confirmed by HPLC or sandwich type ELISA in which two polyclonal antibodies labeled with beta-galactosidase are bonded to an antigen (elcatonin) and measured.

Measurement (Randall Selitto method) by an analgesic effect measuring apparatus against mechanical stimulation was carried out according to a manual of Analgesy meter (UGO BASILE: 7200) . Immediately after initiation of administration, the position of a cage was moved randomly using random number by a person other than a withdrawal latency measuring staff, further, an identification seal on the cage was peeled, thus, measurement was carried out under condition wherein the measuring staff could not identify the individual rats.

First, the position of a pressure needle was controlled to confirm that an arm was horizontal to the thickness of a rat leg. A rat right posterior limb was placed between a pedestal and a pressure needle at the arm end, then, a moving matter was set at 0, a foot pedal was trodden, and the screw rod was rotated and the moving matter was moved at a rate of 16 mm/second, and a load (16 g/second) was applied to the pressure needle. When the rat withdrew the limb, the foot pedal was released, and a numerical value (0 to 25) of the moving matter on a scale was read. The read numerical value was multiplied by 20, to convert into pressed weight (withdrawal threshold). The average value of two measurement values was used as the withdrawal threshold of an individual rat.

In statistical analysis of the result, for confirmation of lowering of the threshold in the CCI vehicle administration group, a value before the operation and the threshold on 9th day after the operation were checked by the paired t-test (SAS system, version 8.2), and a significance of 5% or less was considered to be statistically different (** in a figure: p < 0.01). Comparison of the CCI vehicle administration group and the CCI ECT administration group was analyzed by Williams test, and a hazard ratio of 2.5% or less was judged to be significant (only CCI ECT 0.75 mU/kg group is shown in a figure, #: p < 0.025, ##: p < 0.005).

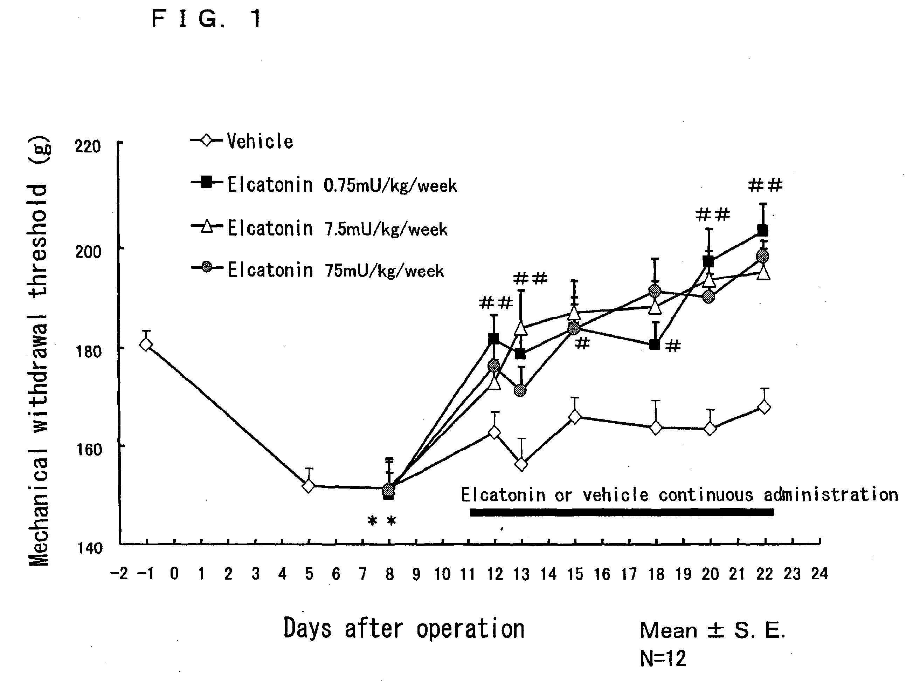

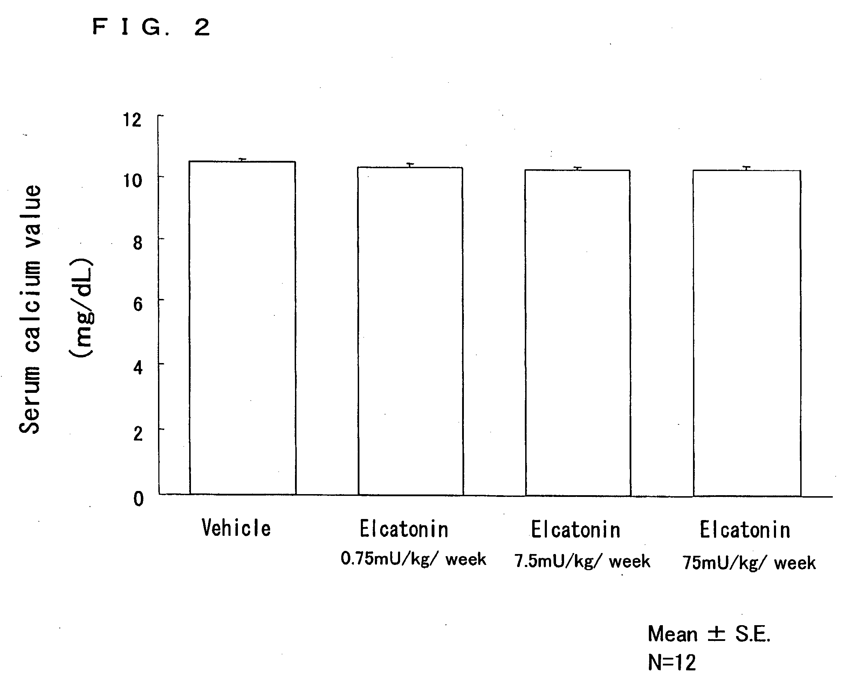

The results are shown in Fig. 1. A significant analgesic action was recognized from the next day after the initiation of the administration (16 hours after), and continued to completion of the test. Further, on 14th day after the initiation of the administration, serum calcium concentration was measured, as a result, as shown in Fig. 2, elcatonin continuous administration did not affect serum calcium amount at all. In the elcatonin continuous administration group, no change in body weight was found as compared with the vehicle administration group.

Therefore, it was clarified that elcatonin continuous administration shows a quick-acting excellent analgesic action for lowering of the nociceptive threshold of a neuropathic pain model.

### Example 2

In the same manner as in Example 1, an analgesic action was examed at elcatonin dosage 750 µU/kg/week, 75 µU/kg/week and 7.5 µU/kg/week.

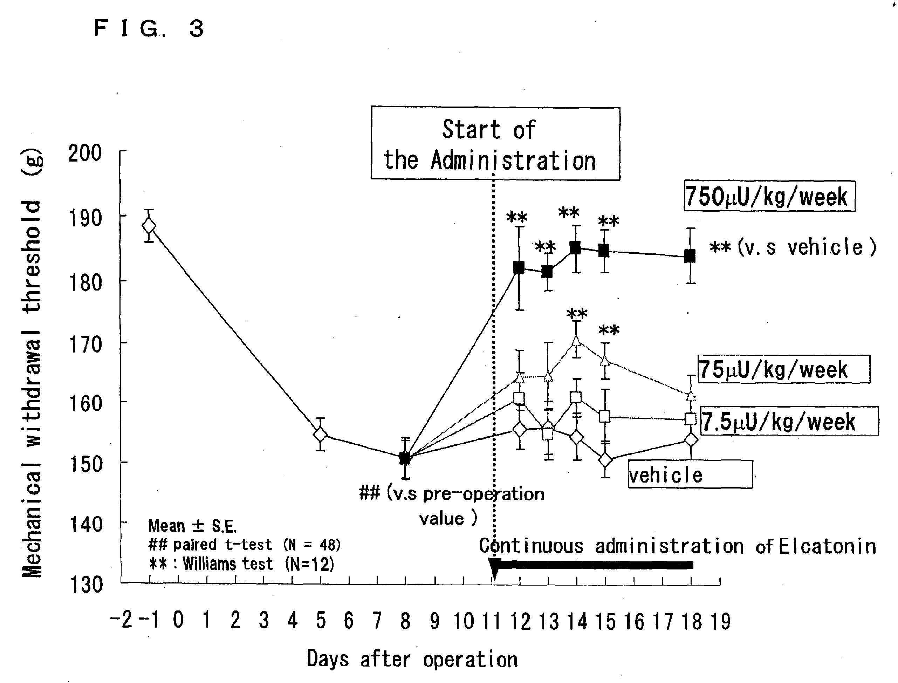

The result is shown in Fig. 3. In the CCI model, significant lowering of nociceptive threshold was recognized as compared with that before the operation (##: p < 0.01) . From the next day after the initiation of the administration, a significant analgesic action was recognized in the 750 µU/kg/week administration group, however, in the 75 µU/kg/week administration group, a significant effect was recognized only on 3rd day and 4th day after administration (**: p < 0.005). In the 7.5 µU/kg/week administration group, a significant effect was not recognized. Therefore, the threshold of the calcitonin dosage exhibiting the analgesic action in a neuropathic pain rat was considered to be 75 µU/kg/week.

### Example 3

Using the same CCI model as in Example 1, an effect of elcatonin continuous administration on hyperalgesia occurring in the CCI model was investigated. Preparing of the CCI model, elcatonin administration method and administration initiation period were the same as in Example 1. The elcatonin dose was 0.00075 U/kg/week, 0.0075 U/kg/week, 0.075 U/kg/week, 7.5 U/kg/week or 75 U/kg/week, and a vehicle was administered in the CCI control group. Measurement was carried out according to a manual of "BASILE Plantar Test" (UGO BASILE: 7370). An apparatus measures a time (withdrawal latency) until escaping after giving nociceptive heat stimulus to a posterior limb in a rat under unrestrained condition. Right hindlimb was measured as a measurement leg. Initially, a rat was placed in an acryl box on a glass plate, and domesticated for about 5 minutes. Amoving type I.R (infrared) generator (I. R. vessel) was placed under the glass plate, and "sight" cross (I.R. irradiation position) inscribed on an upper panel of the I.R. vessel was adjusted to inside of six pads on the right hind paw. In this procedure, contact of the glass plate and hind paw was confirmed. Subsequently, a start key was pushed and heat stimulation was imparted, and withdrawal latency for escaping behavior of a rat of withdrawing limb was measured. When escaping behavior is caused, a switch is automatically turned off, and reaction time is counted. In this measurment, stimulation of I.R. strength of 80 was used, and cut-off time was 22.5 seconds (regulated value). Measurement was carried out under clean condition so that there was no feces and no urine on the glass plate. The mean of two measurement values was used as the withdrawal latency of an individual rat.

In statistical analysis of the result, for confirmation of hyperalgesia (shortening of withdrawal latency) in the CCI vehicle administration group, a value before the operation and the threshold on 9th day after the operation were checked by the paired t-test (SAS system, version 8.2), and a significance of 5% or less was considered to be significant. Comparison of the CCI vehicle administration group and the CCI ECT administration group was analyzed by Williams test, and asignificance of 2.5% or less was considered to be significant (shown in a figure, ##: p < 0.005).

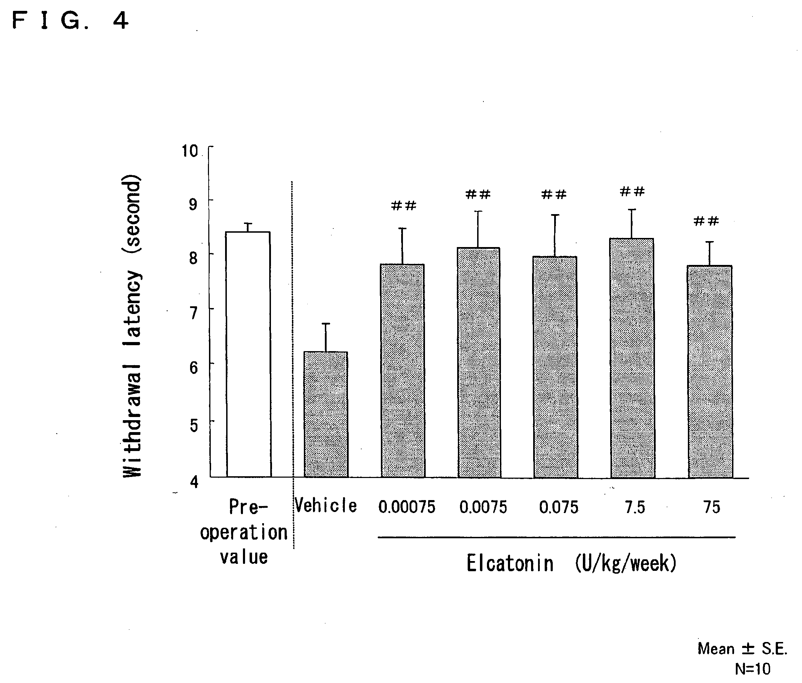

The withdrawal latency on the next day after the initiation of the administration (on 12th day after operation) was shown in Fig. 4. A value before the operation is also shown as a reference in Fig. 4. A significant analgesic action was confirmed in all elcatonin administration groups as compared with the vehicle group. Administration was continued for 2 ensuring weeks, to find continuance of an analgesic action.

Further, on 14th day after initiation of administration, serum calcium concentration was measured, to find that elcatonin continuous administration did not affect serum calcium amount at all.

Therefore, it was clarified that a quick acting excellent analgesic action is shown also on hyperalgesia of a neuropathic pain model. Further, in these dose ranges, extent of an analgesic action was approximately constant, and hypoalgesia over a normal value did not occur.

### Example 4

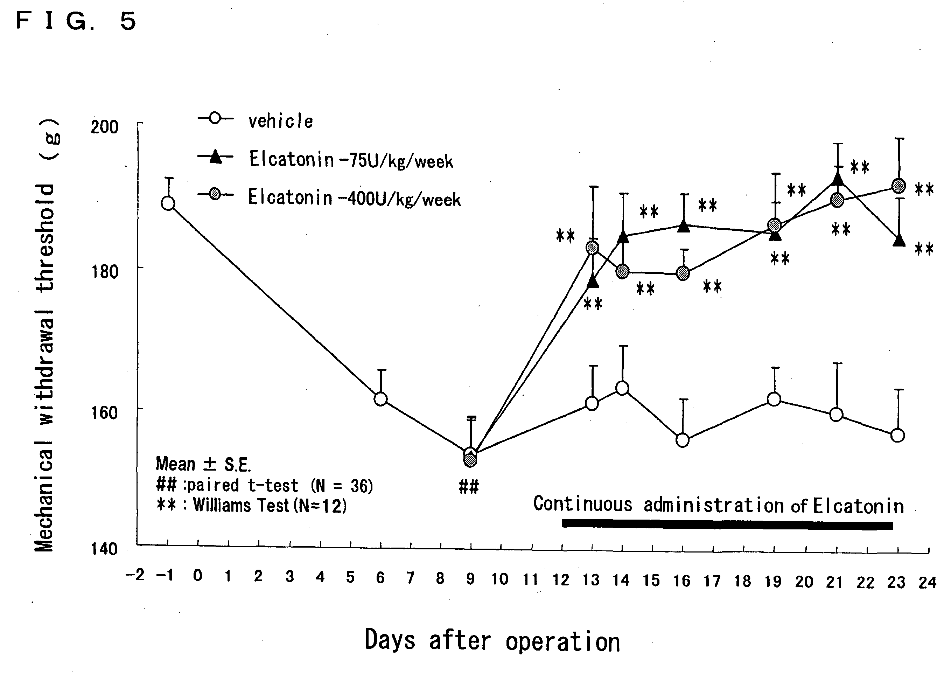

According to the same manner as in Example 1, an analgesic action was checked at elcatonin dose 75 U/kg/week and 400 U/kg/week. The result is shown in Fig. 5. In the CCI model, significant lowering of nociceptive threshold was recognized as compared with that before the operation (##: p < 0.01). From the next day after initiation of administration, a significant analgesic action was recognized, and this effect continued until 11-th day (**: p < 0.005).

Separately, elcatonin of 75 U/kg/week or 400 U/kg/week was administered to rats of the same week old (n = 3), and serum calcium was measured 30 minutes, 60 minutes, 3 hours, 6 hours, 1 day, 4, 6, 8, 12, and 15 days after administration, however, no change was observed as compared with that before administration.

### Example 5

Using the same CCI model as in Example 1, a manifestation time of an analgesic effect by dip intravenous administration was investigated,

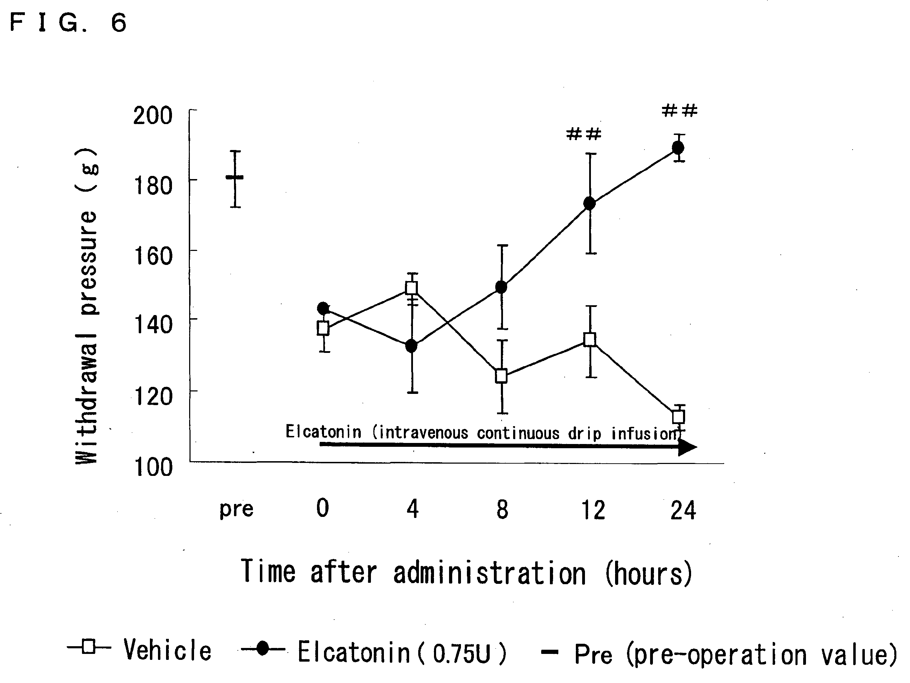

Preparing of the CCI model and elcatonin administration initiation period were the same as in Example 1. A cannulation was performed on cervical vein, and on 2nd day after cannulation, elcatonin (0.75 U/kg/week) was administered by intravenous continuous drip at 1 mL/body/24 hours, and withdrawal pressure by Randall Slitto method was measured 4, 8, 12 and 24 hours after. As a result, on 4 hours, there was no effect, on 8 hours, an improvement tendency was observed, and on 12 hours or more, a significant analgesic action was observed (Fig. 6). Therefore, the threshold of effect manifestation time can be estimated to be 8 hours. After manifestation of an analgesic action, the drug was withdrawn, then, a significant effect continued for 7 days, however, on 10th day after drug withdrawal, pain recurred.

### Preparation Example 1

2 g of poly(lactic-co-glycolic acid) having a molecular weight of about 20000 (PLGA-7520; manufactured by Wako Pure Chemical Industries Ltd., lactic acid:glycolic acid polymerization ratio = 75:20) was dissolved in 100 mL of methylene chloride. 40 mg of elcatonin was dissolved in 150 mL of 0.01 N hydrochloric acid and 100 mL of 1/15 M disodium hydrogenphosphate. Into the elcatonin solution was dropped the PLGA solution, and the mixture was stirred (25000 rpm, 5 min) by a high speed emulsifier (POLYTRON). Centrifugal separation was performed at 4°C, 20000 rpm for 10 minutes, and the supernatant was removed. 2 mL of 1/75M disodium hydrogen phosphate and 200 mL of methylene chloride were added, and the half amount thereof was used in the following processes. This solution was dropped (4 mL/min) into 300 mL of caprylic/capric triglyceride (Triester F810; manufactured by Nikko Chemicals) containing 2% polyglyceryl-6 polyricinoleate (Hexaglyn PR-15; manufactured by Nikko Chemicals)/200 mL of hexane and stirred at 5000 rpm. Under reduced pressure, the mixture as stirred at 37°C overnight and the solvent was distilled off. 200 mL of hexane was added, and centrifugation (4°C, 20000 rpm, 10 min) was performed, and the supernatant was removed. 160 mL of hexane was added and centrifugal separation was performed in the same manner, and the supernatant was removed. 80 mL of 2% polyvinyl alcohol (PVA-403; manufactured by Kuraray Co. Ltd.) solution was added, and centrifugal separation (4°C, 20000 rpm, 10 min) was performed, and the supernatant was removed. 20 mL of purified water was added, and particle size was measured using Zetasizer 3000HS (Malvern Instruments Ltd, Malvern UK). Calculation of particle size was carried by a Cumulant method from self correlation coefficient obtained by dynamic light scattering (photon correlation method), and its average particle size (ZAve) was 315.7 nm.

Measurement of content ratio: About 30 mg of elcatonin-PLGA micro-spheres was weighed precisely, and put into a 5 mL measuring flask. To this was added 0. 5 mL of purified water and suspension was made again, 3. 5 mL of acetone was added to dissolve PLGA. The volume was increased to 5 mL with purified water to cause deposition of PLGA. This deposit was precipitated by centrifugal separation (15°C, 20000 rpm, 15 min), and the supernatant was used as a sample solution, and analyzed by HPLC in comparison with an elcatonin standard solution. As a result, the elcatonin-PLGA micro-sphere had an elcatonin content of 3.4 µg/mg.

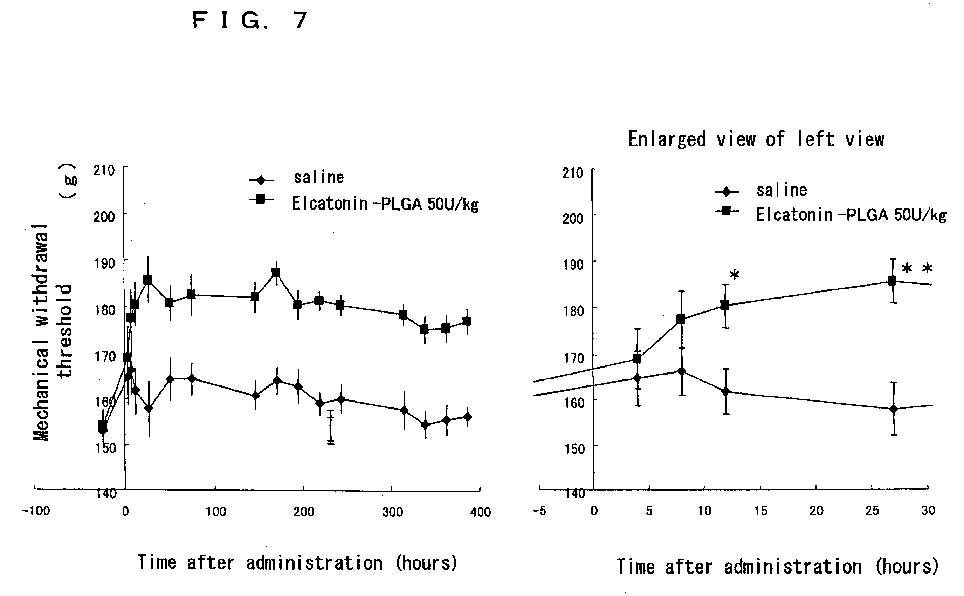

Analgesic experiment: The same CCI model as in Example 1 was used, and an effect of the elcatonin-PLGA micro-sphere on hyperalgesia in the CCI model was investigated. The elcatonin-PLGA micro-sphere was dissolved in saline to give 50 U/mL. In an animal showing occurrence of hyperalgesia, the prepared solution was administered subcutaneously at neck at a rate of 1 mL/kg. 4, 8, 12 and 24 hours after administration, withdrawal pressure by Randall Selitto method was measured once a day (from Monday to Friday) further until 387 hours after. As a result, on 4 hours, there was no effect, on 8 hours, an improvement tendency was observed, and on 12 hours or more, a significant analgesic action was observed, and its effect continued until 387 hours after (Fig. 7). Therefore, the threshold of effect manifestation time can be estimated to be 8 hours.

Blood elcatonin concentration and serum calcium concentration were measured using a normal rat of the same week old as the CCI model. As a result, the blood elcatonin concentration was high within one day directly after administration, recognizing so-called initial period burst. Thereafter, until 387 hours after, the concentration was maintained in a range of 1.3 to 3.9 pg/mL. On the other hand, the serum calcium concentration lowered transiently, but did not decrease below a normal range, and on 24 hours, the concentration returned to a value before administration, and thereafter, no change was observed until 387 hours after.

### Comparative Example 1

An action of intermittent administration of elcatonin was investigated in the same manner as in Example 1.

From two weeks after in coming until initiation of elcatonin (ECT) administration, a vehicle (0.02% BSA-containing 0.1 mM sodium acetate buffer (pH 5.5)) was administered subcutaneously 5 times a week at 0.05 ml/B.W.100g to all animals. Using withdrawal threshold and body weight on 9th day after the constriction operation as indices, rats were distributed into a CCI-vehicle group (12 individuals), and CCI-ECT 15 U/kg/3 times a week (Monday, Wednesday, Friday; vehicle for Tuesday and Thursday) and CCI-ECT 15 U/kg/5 times a week (Monday, Tuesday, Wednesday, Thursday, Friday) groups (each 12 rats), by a randomization method (SAS system, version 8.2). On the 12th day or later after the constriction operation, ECT or vehicle was administered subcutaneously at 0.05 ml/B.W.100 g over a period of 10 seconds per one shot.

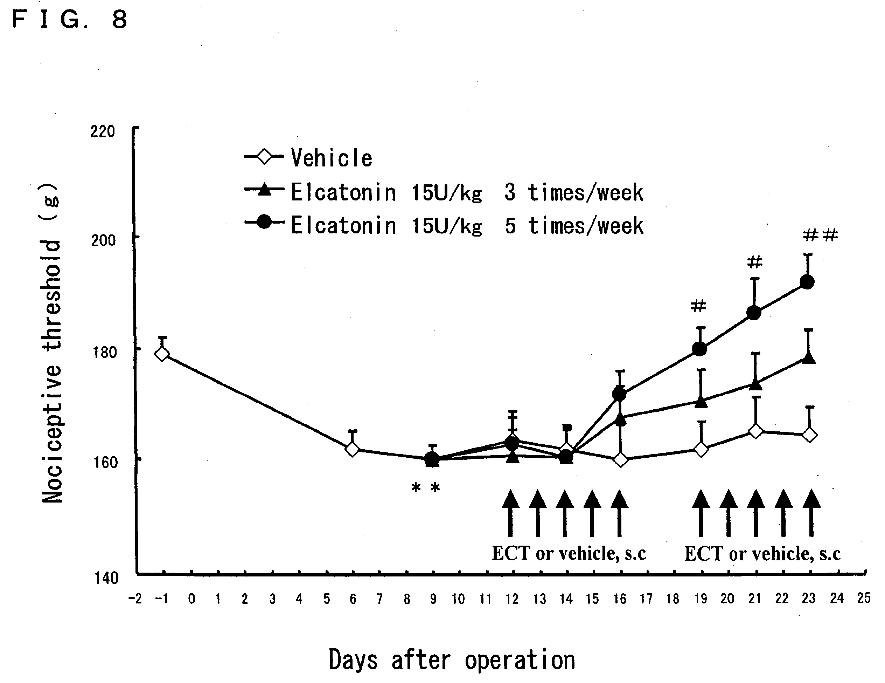

The result is shown in Fig. 8. In the CCI model, a significant lowering of nociceptive threshold was recognized as compared with that before the operation (**: p < 0.01). Until 3-rd day after initiation of ECT administration, an effect was not observed, while in the ECT 5 times a week administration group, from 4th day after initiation of administration, the threshold initiated to recover gently, and on 7th day or later, a significant analgesia action was recognized (#: p < 0.025, ##: p < 0.005). In the ECT 3 times a week administration group, an improvement tendency was observed, however, a significant action was not observed. In the ECT administration group, suppression of body weight increase was recognized. Separately, the dose was further increased and a test was conducted, as a result, delay of manifestation of an analgesic effect was not improved, and suppression of body weight increase became remarkable, thus, it was considered that this is not suitable as a therapeutic medicine.

Therefore, it could be confirmed that elcatonin intermittent administration improves neuropathic pain, but its efficiency is delayed and a satisfactory treatment effect is not obtained. Both in the CCI-ECT 15 U/kg 3 times a week administration group and the CCI-ECT 15 U/kg 5 times a week administration group, variation in serum calcium value was observed.

### Comparative Example 2

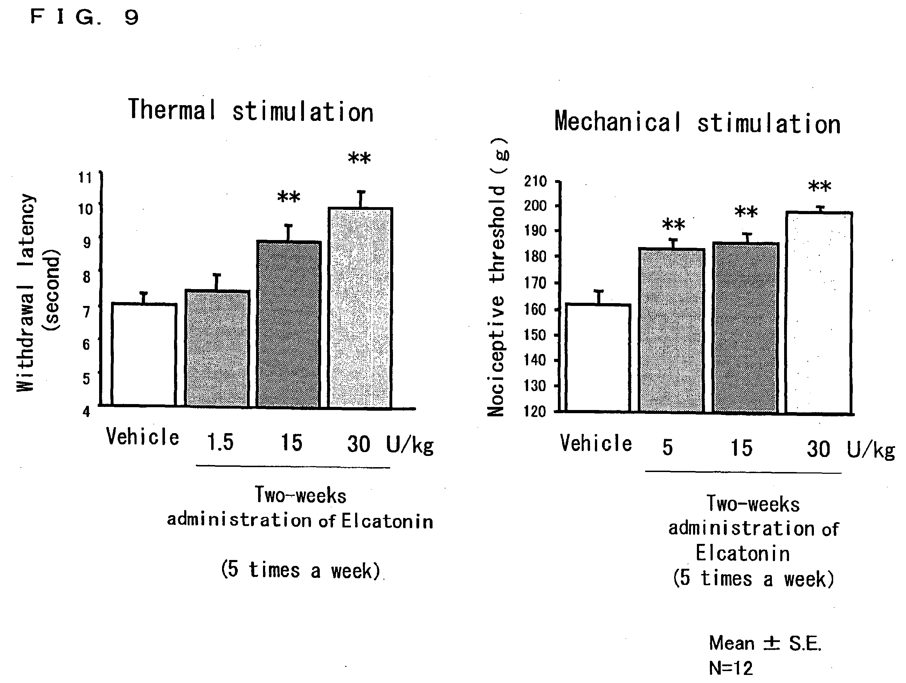

According to the same manner as in Comparative Example 1, dose dependency of elcatonin 5 times a week intermittent subcutaneous administration was investigated. The result after administration for 2 weeks is shown in Fig. 9. For lowering of nociceptive threshold by Randall Selitto method, an effect was observed in administration of 5 U/kg, and for thermal hyperalgesia, an effect was not observed at 1.5 U/kg, and an effect was observed at 15 U/kg or more. From the result of Test Example 1 described below, it was clarified that with effective dose on neuropathic pain in intermittent administration, serum calcium is lowered to normal value (8.5 to 10.5 mg/dl) or less.

### Test Example 1

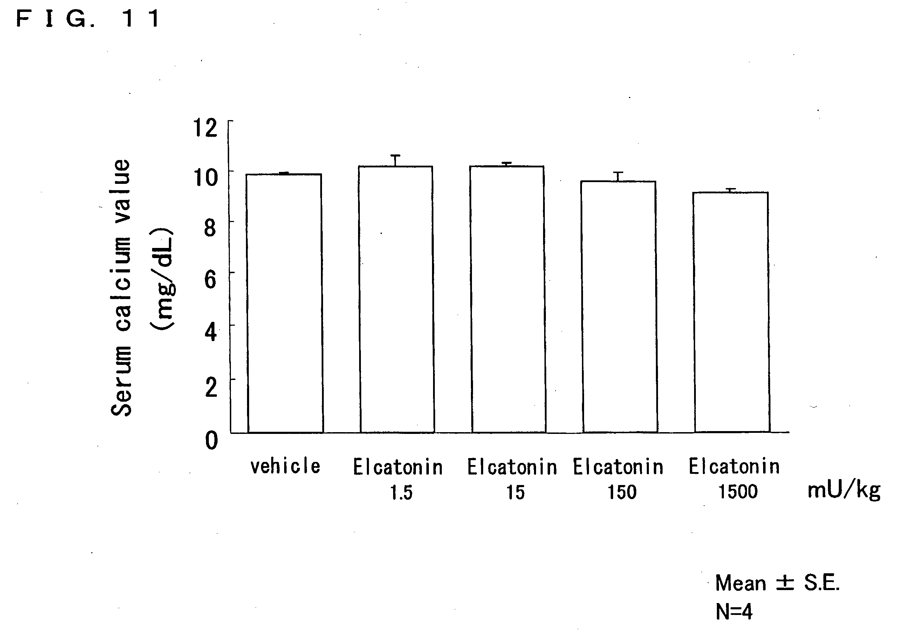

Using normal rats (each 4 rats) which were in the same week old in initiation of administration of elcatonin as in Examples 1 and 2, dose of elcatonin not varying serum calcium value was examed in the case of single subcutaneous administration (administered over 10 seconds). Timing of blood collection was 60 minutes after administration at which serum calcium value of a rat by elcatonin was maximum. Further, the same test was conducted excepting that blood collection was carried out 30 minutes after the administration. Measurement of serum calcium value was conducted using calcium C-test wako (Wako). As shown in Figs. 10 and 11, serum calcium value were changed at 150 mU/kg or more, namely, at 15 mU/kg/second or more per unit time (second), in single subcutaneous injection. This serum calcium value was below a normal value.

From the above-mentioned results, it was confirmed that a calcitonin continuous administration preparation in neuropathic pain is a useful agent for treatment and/or prevention, which manifests an excellent action even at lower dosage as compared with intermittent administration, and can reduce side effects and dosage frequency.

### Industrial Applicability

The present invention shows an effective action on neuropathic pain and is suitable as a medicine.

### Brief Explanation of Drawings

Fig. 1 shows change in nociceptive threshold in continuous administration of elcatonin against lowering of nociceptive threshold of sciatic nerve constriction model.
   **: comparison of nociceptive threshold before and after a constriction operation by t-test p < 0.01
   #: comparison of nociceptive threshold of a vehicle administered group and an elcatonin 0.75 mU/kg/week administered group by Williams test p < 0.025
   ##: comparison of nociceptive threshold of a vehicle administered group and an elcatonin 0.75 mU/kg/week administered group by Williams test p < 0.005
Fig. 2 shows serum calcium concentration after continuous administration of elcatonin for 14 days (after completion of the test in Fig. 1) on a sciatic nerve constriction model.
Fig. 3 shows change in nociceptive threshold in continuous administration of elcatonin against lowering of nociceptive threshold of a sciatic nerve constriction model.
   ##: comparison of nociceptive threshold before and after a constriction operation by t-test p < 0.01
   **: comparison of nociceptive threshold of a vehicle administered group and an elcatonin administered group by Williams test p < 0.005
Fig. 4 shows withdrawal latency at the next day after initiation of administration in continuous administration of elcatonin for hyperalgesia of a sciatic nerve constriction model. A value before an operation is also shown for reference.
   ##: comparison of withdrawal latency of a vehicle administered group and an elcatonin administered group by Williams test p < 0.005
Fig. 5 shows change in nociceptive threshold in continuous administration of elcatonin against lowering of nociceptive threshold of a sciatic nerve constriction model.
   ##: comparison of nociceptive threshold before and after an operation by t-test p < 0.01
   **: comparison of nociceptive threshold of a vehicle administered group and an elcatonin administered group by Williams test p < 0.005
Fig. 6 shows nociceptive threshold in intravenous continuous drip administration of elcatonin against hyperalgesia of a sciatic nerve constriction model. A value before an operation (pre) is also shown for reference.
   ##: comparison of nociceptive threshold of a vehicle administered group and an elcatonin administered group by t-test p < 0.01
Fig. 7 shows nociceptive threshold in administration of elcatonin-PLGA micro-sphere (EL-PLGA) against hyperalgesia in a sciatic nerve constriction model. A right view is an enlarged view of the left view. Only in the right view, a statistical significant difference is represented by the following mark.
   * or **: comparison of nociceptive threshold of saline administered group and a EL-PLGA administered group by t-test p < 0.05 or p < 0.01
Fig. 8 shows change in nociceptive threshold in intermittent administration of elcatonin using a sciatic nerve constriction model. An arrow shows a day on which elcatonin is subcutaneously administered intermittently.
   **: comparison of nociceptive threshold before and after a constriction operation by t-test p < 0.01
   #: comparison of nociceptive threshold of a vehicle administered group and an elcatonin administered group by Williams test p < 0.025
   ##: comparison of nociceptive threshold of a vehicle administered group and an elcatonin administered group by Williams test p < 0.005
Fig. 9 shows nociceptive threshold or withdrawal latency after two-weeks administration in intermittent administration of elcatonin against lowering of hyperalgesia or nociceptive threshold of a sciatic nerve constriction model.
   **: comparison of withdrawal latency of a vehicle administered group and an elcatonin administered group by Williams test p < 0.005
Fig. 10 shows change in serum calcium concentration in single subcutaneous administration of elcatonin in a normal rat (60 minutes after administration).
Fig. 11 shows change in serum calcium concentration in single subcutaneous administration of elcatonin in a normal rat (30 minutes after administration).

## Claims

1. An agent comprising calcitonin as an active ingredient for use in a method of treating and/or preventing neuropathic pain, wherein calcitonin is continuously administered in an amount less than 0.093 mU/kg/second.

2. The agent for use as in claim 1, wherein the duration of the continuous administration is 8 hours or more.

3. The agent for use as in claim 1 or 2, wherein the neuropathic pain is caused by the compression of the peripheral nerve or the injury of the peripheral nerve.

4. The agent for use as in any of claims 1 to 3, wherein the calcitonin is elcatonin.

5. The agent for use as in any of claims 1 to 4, wherein the dosage form is a sustained release preparation, a drip preparation or a patch preparation.

6. The agent for use as in claim 5, wherein the dosage form is an injection preparation, an implanted preparation, a percutaneous preparation, a transnasal preparation, a transpulmonary preparation or a peroral preparation.

## Patentansprüche

1. Mittel, das Calcitonin als einen Wirkstoff umfasst, zur Verwendung bei einem Verfahren zur Behandlung und / oder Vorbeugung von neuropathischem Schmerz, wobei Calcitonin kontinuierlich in einer Menge von weniger als 0,093 mU/kg/Sekunde verabreicht wird.

2. Das Mittel zur Verwendung wie in Anspruch 1, wobei die Dauer der kontinuierlichen Verabreichung 8 Stunden oder mehr beträgt.

3. Das Mittel zur Verwendung wie in Anspruch 1 oder 2, wobei der neuropathische Schmerz durch die Kompression eines peripheren Nervs oder die Schädigung eines peripheren Nervs verursacht wird.

4. Das Mittel zur Verwendung wie in einem der Ansprüche 1 bis 3, wobei das Calcitonin Elcatonin ist.

5. Das Mittel zur Verwendung wie in einem der Ansprüche 1 bis 4, wobei die Darreichungsform eine Zubereitung mit verzögerter Freisetzung, eine Tropf- Zubereitung oder eine Pflaster-Zubereitung ist.

6. Das Mittel zur Verwendung wie in Anspruch 5, wobei die Darreichungsform eine Injektions- Zubereitung, eine implantierte Zubereitung, eine perkutane Zubereitung, eine transnasale Zubereitung, eine transpulmonale Zubereitung oder eine perorale Zubereitung ist.

## Revendications

1. Agent comprenant de la calcitonine en tant que principe actif pour son utilisation dans un procédé de traitement et/ou de prévention de la douleur neuropathique, dans lequel la calcitonine est administrée en continu en une quantité inférieure à 0,093 mU/kg/seconde.

2. Agent pour son utilisation selon la revendication 1, dans lequel la durée de l'administration continue est de 8 heures ou plus.

3. Agent pour son utilisation selon la revendication 1 ou la revendication 2, dans lequel la douleur neuropathique est entraînée par la compression du nerf périphérique ou la lésion du nerf périphérique.

4. Agent pour son utilisation selon l'une quelconque des revendications 1 à 3, dans lequel la calcitonine est l'elcatonine.

5. Agent pour son utilisation selon l'une quelconque des revendications 1 à 4, dans lequel la forme posologique est une préparation à libération prolongée, une préparation pour perfusion ou une préparation pour timbre.

6. Agent pour son utilisation selon la revendication 5, dans lequel la forme posologique est une préparation pour injection, une préparation à implanter, une préparation percutanée, une préparation transnasale, une préparation transpulmonaire ou une préparation perorale.
